# EUROPEAN PATENT APPLICATION

(11) **EP 4 483 926 A1**
(43) Date of publication of application: **01.01.2025**
(21) Application number: 23777972.3
(22) Date of filing: 23.03.2023
(51) Int. Cl.: A61M 5/20

(54) **AUTOMATIC INJECTION PEN**

(30) Priority: 30.03.2022 CN 202220737436 U; 21.03.2023 CN 202310279336
(71) Applicant: Suzhou Savicred Biotechnology Co., Ltd., Suzhou, Jiangsu 215000 (CN)
(72) Inventor: LIN, Xiao, Suzhou, Jiangsu 215000 (CN); ZOU, Hongxia, Suzhou, Jiangsu 215000 (CN); HU, Guangjun, Suzhou, Jiangsu 215000 (CN)
(74) Representative: Vitina, Maruta
(86) International application number: PCT/CN2023/083222
(87) International publication number: WO 2023/185602

(57) **Abstract**

An automatic injection pen includes a shell, a needle cover slidably provided in the shell, an inner sleeve, a pen cap, a spring cylinder, a pen tube stopper and a syringe assembly. The syringe assembly includes a barrel, a needle and a piston assembly. The piston assembly includes a push head, a push rod, a spring support portion, a locking protrusion, a guiding portion and an actuation portion from bottom to top. An outer side of the spring support portion is provided with a spring for drug administration. The piston assembly, the spring, the spring cylinder, the needle cover, and the pen tube stopper constitute a locking-actuation mechanism. A push rod of the piston assembly is provided with an elastic protrusion structure. A linear buffer is provided between the push head and a piston in the barrel to delay the needle withdrawal.

## Description

### TECHNICAL FIELD

This application relates to medical injection appliances, and more particularly to an automatic injection pen.

### BACKGROUND

Chinese Patent Application Publication No. 113499513A disclosed an automatic injection pen, where by means of the cooperation of an automatic injection mechanism and a spring, the whole process of insertion, drug injection and withdrawal can be completed by a single pressing operation, that is, the push-out of a disposable syringe from the pen body, drug administration, and withdrawal and covering of needle can be simply completed, thereby making the drug injection more reliable.

However, the above automatic injection pen still has the following disadvantages in the actual operation process.
(1) There is a lack of a structure to lock the actuation mechanism, resulting in a risk of accidental release of the injection pen during the transportation process.
(2) Some connection structures are unreliable or are difficult to manufacture.
(3) Some operations cannot be completely performed, such as incomplete drug administration, affecting the use experience.

Therefore, it is necessary to further improve the automatic injection pen to arrive at one with reliable structure and complete drug delivery, which satisfies the storage, transportation and use needs of the medicament in the disposable syringe.

### SUMMARY

An object of this application is to provide an automatic injection pen with enhanced structure reliability and complete drug delivery.

This application provides an automatic injection pen, comprising:
a shell;
a needle cover;
an inner sleeve; and
a syringe assembly;
wherein the needle cover is slidably provided in the shell; a top of the needle cover is provided with a first opening; an inner side of a lower part of the needle cover is fixedly provided with a spring sleeve; an upper part of the spring sleeve is provided with a spring hook configured to protrude inwardly; the inner sleeve is slidably arranged in the needle cover; a top of the inner sleeve is provided with a second opening; an outer side of a lower part of the inner sleeve is provided with a sliding groove; a bottom of the sliding groove is closed, and an upper part of the sliding groove is provided with a first protrusion; the spring hook is configured to be clamped in the sliding groove; a first protrusion is provided between the lower part of the inner sleeve and the spring sleeve for needle withdrawal; the syringe assembly comprises a barrel, a needle, and a piston assembly; the barrel is arranged in the inner sleeve; the needle is arranged at the a bottom of the barrel, and the piston assembly is provided in an upper part of the barrel; the piston assembly comprises a push head, a push rod, a spring support portion, a second protrusion, a guiding portion and an actuation portion sequentially from bottom to top; an outer side of the push rod is provided with a withdrawal-triggering claw; the second protrusion is configured to be pre-clamped in the second opening; an outer side of the spring support portion is provided with a second spring; the second spring is configured to be pre-compressed to abut against an inner side of the top of the inner sleeve; the guiding portion is configured to pass through the first opening; the lower part of the inner sleeve is provided with a third opening configured for the withdrawal-triggering claw to pass through; and the withdrawal-triggering claw is located above the third opening, and is located above the spring hook;
wherein the automatic injection pen further comprises a spring cylinder and a pen tube stopper; and the piston assembly, the second spring, the spring cylinder, the needle cover, and the pen tube stopper constitute a locking-actuation mechanism;
an upper part of the piston assembly comprises the spring support portion; the spring support portion is a pair of elastic claws; an outer side of the spring support portion is configured to accommodate the second spring; and an upper part of the spring support portion is provided with a locking-actuation structure;
the locking-actuation structure comprises a spring locking groove, a piston locking groove and an actuation column;
the spring locking groove is located on a middle-upper part of the spring support portion; the piston locking groove is located on the upper part of the spring support portion; and the actuation column is located on an outer side of the middle-upper part of the spring support portion;
the second spring is a compression spring; and the spring cylinder is provided on an outer side of the second spring;
an upper end of the second spring abuts against an inner side of a top of the spring cylinder, and a lower end of the second spring abuts against a bottom platform of the spring support portion;
the top of the spring cylinder is provided with a piston actuation channel; and an inner side of the piston actuation channel is provided with a spring locking structure;
the needle cover is sleeved outside the spring cylinder, and is configured to axially move;
the top of the needle cover is provided with a fourth opening configured for a top of the piston assembly to pass through; and an inner side of the needle cover is provided with a guide plate;
an inner side of the guide plate is provided with a slope;
an outer side of the needle cover is provided with the pen tube stopper;
an interior of the pen tube stopper is provided with a locking claw; and a bottom of the locking claw is inwardly provided with a locking hook;
the locking-actuation mechanism is configured to have a locking state and an actuation state;
in response to the locking state of the locking-actuation mechanism:
   the upper part of the spring support portion is configured to pass through the piston actuation channel and the fourth opening; the piston locking groove is configured to be clamped into the locking claw, and to be engaged with the locking hook;
   the middle-upper part of the spring support portion is configured to be clamped on the top of the spring cylinder; the spring locking groove is configured to be clamped in the spring locking structure of the spring cylinder;
   the second spring is in a limited compression state; and
   the needle cover is located at a position corresponding to a lower part of the shell; the guide plate is located on the outer side of the spring support portion; and the slope is located below the actuation column;
   in response to the actuation state of the locking-actuation mechanism:
      the needle cover is located at a position corresponding to an upper part of the shell; the guide plate is configured to move upward along the outer side of the spring support portion; the slope is in contact with the actuation column; the actuation column is configured to be guided to move inward to a bottom free space of the slope to drive the upper part of the spring support portion to contract inward;
      the piston locking groove is configured to move inward to be separated from the locking hook;
      the spring locking groove is configured to move inward to be separated from the spring locking structure; and
      the second spring is in an elastic release state;
      a part of the push rod within the barrel is provided with a third protrusion; and both sides of the piston assembly are symmetrically provided with the third protrusion;
      a linear buffer is provided between the push head of the piston assembly and a piston in the barrel;
      an inner support for the withdrawal-triggering claw is provided in the third opening; and the inner support is connected with the first protrusion; and
      an outer surface of the claw inner support is below an outer surface of the first protrusion.

In an embodiment, the number of the guide plate is two; two guide plates are symmetrically arranged at an inner bottom of the fourth opening;
the number of the slope is four, and each of the two guide plates is symmetrically provided with two of four slopes;
the number of the actuation column is four; the number of the spring support portion is two, and two spring support portions are respectively provided at two sides of the piston assembly; and four actuation columns are respectively located on both sides of the two spring support portions; and
the four actuation columns are respectively configured to cooperate with the four slopes. In an embodiment, a first connecting plate is provided between the two guide plates.

In an embodiment, a clamping column is provided in the fourth opening; and the number of the spring support portion is two, and two spring support portions are respectively provided at two sides of the piston assembly;
in a case that the locking-actuation mechanism is in the locking state, the clamping column is located between the spring support portion; and
in a case that the locking-actuation mechanism is in the actuation state, the clamping column is located above the two spring support portions.

In an embodiment, a second connecting plate is provided between the clamping column and the guide plate.

In an embodiment, a middle inner side of the upper part of each of the two spring support portions is provided with a guide groove; and
the guide groove is configured to fit the clamping column.

In an embodiment, a limit claw is provided on an outer side of the locking claw; and the limit claw is a straight blocking plate;
two sides of the piston locking groove are each provided with a limit surface; and
the limit surface is configured to fit with the limit claw.

In an embodiment, a connecting plate channel is provided at a middle of the limit claw.

In an embodiment, a limit plate is provided in the spring locking groove; and a middle of the spring locking structure is provided with a limit groove; and
the limit plate is configured to be clamped into the limit groove in response to a case that the spring locking groove is engaged with the spring locking structure.

In the locking-actuation mechanism of the present disclosure, the needle cover is the only actuation component; and the needle cover is configured to move upward to actuate.

In an embodiment, the third protrusion and the piston assembly are integrally formed;
an outer diameter of the third protrusion is larger than an inner diameter of the barrel; and
an inner side of the third protrusion is provided with a cavity; and the third protrusion has a transverse elasticity.

In an embodiment, the linear buffer comprises a case and a buffer plug;
a bottom of the case is connected with the piston; and a top of the buffer plug is connected with the push head;
a sealing ring is provided between the case and an upper part of the buffer plug for slidable sealing; and
a first buffer cavity is provided between a bottom of the buffer plug and a bottom of an inner cavity of the case;
a middle of the buffer plug is provided with a recessed portion; and a second buffer cavity is provided between the recessed portion and an inner wall of the case;
a connecting channel is provided between the first buffer cavity and the second buffer cavity; and
the first buffer cavity is configured to be injected with a buffer fluid before using.

In an embodiment, a top of the claw inner support is provided with a first guide slope.

In an embodiment, the automatic injection pen further comprises a pen cap; an inner side of the pen cap is provided with a safety sleeve; a plurality of grooves are circumferentially arranged at an upper part of the safety sleeve; and
the upper part of the safety sleeve is in an open state, and is elastic.

In an embodiment, the inner side of the pen cap is provided the safety sleeve, and a lower part of the safety sleeve is provided with a fixing fastener structure;
the lower part of the safety sleeve has a hollow cavity; an elastic claw is provided in the hollow cavity; and a fourth protrusion is provided below the elastic claw;
the number of the elastic claw is two; and two elastic claws are arranged opposite to each other below the safety sleeve;
the fourth protrusion is configured to protrude outward to exceed an outer surface of the safety sleeve;
upper and lower parts of the fourth protrusion are configured to form a second guide slope;
a protruding sleeve is provided below the needle cover; and a fifth protrusion is inward provided on a bottom of the protruding sleeve; and
in response to a case that the pen cap is assembled with a pen body, the fifth protrusion is configured to pass through a highest point above the two elastic claws to arrive at a position below the two elastic claws; and the two elastic claws are configured to be elastically reset, and to abut against the fifth protrusion.

In an embodiment, the spring sleeve is provided with an elastic fixing fastener; three sides of the elastic fixing fastener are hollow; and a bottom of the elastic fixing fastener is connected with a main body of the spring sleeve;
an upper part of the elastic fixing fastener is configured as a support and limit part, and is configured to extend into a fifth opening provided at the needle cover in a natural state; and the fifth opening is configured to fix the spring sleeve;
a third guide slope is downward and inward provided below the support and limit part; and
the needle cover is provided with a base support.

Compared with the previous design, the automatic injection pen of the present disclosure has the following improvements.
(1) A structure of the pen cap is improved, which is convenient for assembly, improves the connection reliability before use, and improves the reliability of the process of storage and transportation.
(2) An actuation mechanism is improved, and a locking mechanism in an unused state is added to ensure that the actuation mechanism will not come loose in the unused state, which avoids actuation and medicament release in advance caused by spring actuation.
(3) The push rod of the piston assembly is improved, and an elastic structure is added, so that the drug injection is performed after the needling, and the actions after release of the second spring are conducted in an obvious sequence, which improves the injection safety.
(4) The linear buffer is provided between the push head of the piston assembly and the piston in the barrel to delay the needle withdrawal, which ensures complete injection of the medicament, improves utilization rate of the medicament, and has a certain pause time after the injection to satisfy the injection requirements.
(5) The claw inner support is provided in the third opening to support the withdrawal-triggering claw during actuation process, which improves the reliability of a process of withdrawal and actuation.
(6) The connection structure between the needle cover and the spring sleeve is improved, which is convenient for assembly in the process of industrial production, and can perform complete and reliable locking to prevent loose.

The automatic injection pen of the present disclosure has the reliable structure and thorough use of the medicament, which needs of transportation and use of the medicament in the disposable syringe.

### BRIEF DESCRIPTION OF THE DRAWINGS

Fig. 1 shows an overall structure of an automatic injection pen in an unused state according to an embodiment of the present disclosure.
Fig. 2 is a sectional view of the overall structure of the automatic injection pen in the unused state according to an embodiment of the present disclosure.
Fig. 3 is a structural diagram of the automatic injection pen ready for use in a process of removing a pen cap according to an embodiment of the present disclosure.
Fig. 4 is structural diagram of the automatic injection pen ready for use with removal of the pen cap according to an embodiment of the present disclosure.
Fig. 5 shows an internal structure of the pen cap of the automatic injection pen according to an embodiment of the present disclosure.
Fig. 6 is an internal sectional view of the pen cap of the automatic injection pen according to an embodiment of the present disclosure.
Fig. 7 shows a fixing structure of the pen cap of the automatic injection pen according to an embodiment of the present disclosure.
Fig. 8 shows an inner structure of a pen tube stopper according to an embodiment of the present disclosure.
Fig. 9 shows an overall structure of a needle cover according to an embodiment of the present disclosure.
Fig. 10 is an internal sectional view of an upper part of the needle cover according to an embodiment of the present disclosure.
Fig. 11 shows an overall structure of a piston assembly according to an embodiment of the present disclosure.
Fig. 12 is a structural diagram of an upper part of the piston assembly according to an embodiment of the present disclosure.
Fig. 13 is a schematic diagram of an assembly process of the piston assembly and a spring cylinder in a first state according to an embodiment of the present disclosure.
Fig. 14 is a schematic diagram of the assembly process of the piston assembly and the spring cylinder in a second state according to an embodiment of the present disclosure.
Fig. 15 shows an inner structure of the automatic injection pen in a locking state according to an embodiment of the present disclosure.
Fig. 16 shows a partial structure of the automatic injection pen in the locking state according to an embodiment of the present disclosure.
Fig. 17 is a partial diagram of the automatic injection pen in a process of actuation unlocking (unlocked state) according to an embodiment of the present disclosure.
Fig. 18 shows an overall inner structure of the automatic injection pen in the process of actuation unlocking (locked state) according to an embodiment of the present disclosure.
Fig. 19 schematically shows an anti-rotation structure of a spring locking groove according to an embodiment of the present disclosure.
Fig. 20 schematically shows the anti-rotation structure of the spring locking groove according to an embodiment of the present disclosure.
Fig. 21 schematically shows an anti-rotation structure of a piston locking groove according to an embodiment of the present disclosure.
Fig. 22 schematically shows the anti-rotation structure of the piston locking groove according to an embodiment of the present disclosure.
Fig. 23 is a structural diagram of a connecting plate channel according to an embodiment of the present disclosure.
Fig. 24 is a structural diagram of the connecting plate channel according to an embodiment of the present disclosure.
Fig. 25 shows the overall structure of the piston assembly according to an embodiment of the present disclosure.
Fig. 26 schematically shows an assembly state of a push rod in a barrel according to an embodiment of the present disclosure.
Fig. 27 is an assembly diagram of a linear buffer according to an embodiment of the present disclosure.
Fig. 28 schematically shows a specific structure the linear buffer according to an embodiment of the present disclosure.
Fig. 29 is a schematic diagram of a claw inner support according to an embodiment of the present disclosure.
Fig. 30 is a schematic diagram of the claw inner support in a using state according to an embodiment of the present disclosure.
Fig. 31 is a schematic diagram of a connection structure between the needle cover and a spring sleeve according to an embodiment of the present disclosure.
Fig. 32 is a schematic diagram of the connection structure between the needle cover and the spring sleeve according to an embodiment of the present disclosure.
Fig. 33 schematically shows the process of actuation unlocking (unlocked state) in a working process according to an embodiment of the present disclosure.
Fig. 34 schematically shows the process of actuation unlocking (locked state) in the working process according to an embodiment of the present disclosure.
Fig. 35 schematically shows a process of needling in the working process according to an embodiment of the present disclosure.
Fig. 36 schematically shows a process of injection in the working process according to an embodiment of the present disclosure.
Fig. 37 schematically shows a working process of the linear buffer after injection in the working process according to an embodiment of the present disclosure.
Fig. 38 schematically shows a process of needle withdrawal after injection in the working process according to an embodiment of the present disclosure.

In the Figures: barrel 11; needle 12; pen tube body 21; pen tube stopper 22; pen cap 23; needle cover 3; piston assembly 4; second spring 51; first protrusion 52; spring cylinder 6; syringe sleeve 7; spring sleeve 8; and syringe guard ring 9.

### DETAILED DESCRIPTION OF EMBODIMENTS

The embodiments of the present disclosure will be clearly described with reference to the accompanying drawings.

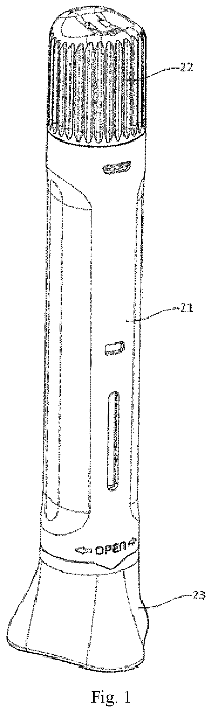

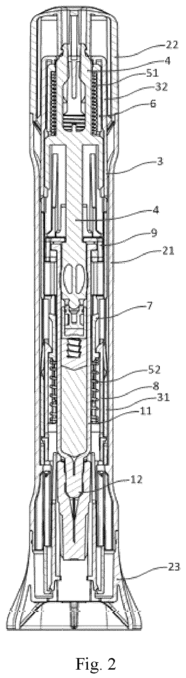

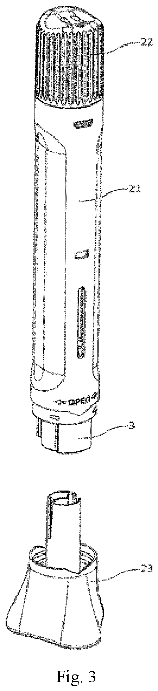

The present disclosure is an improvement of an automatic injection pen of Chinese Patent Application Publication No. 113499513A, and names of components marked in the accompanying drawings of the present disclosure refer to labels in drawings of the Chinese Patent Application Publication No. 1 134995 13 A. Fig. 1 shows an overall structure of an automatic injection pen in an unused state of the present disclosure. Fig. 2 is a sectional view of the overall structure of the automatic injection pen in the unused state. Figs. 3-4 are structural diagrams of the automatic injection pen ready for use. The overall structure of the automatic injection pen of the present disclosure has several improvements.

### Embodiment 1 Improvement of pen cap 23

### (1) Improvement of upper part of safety sleeve 236

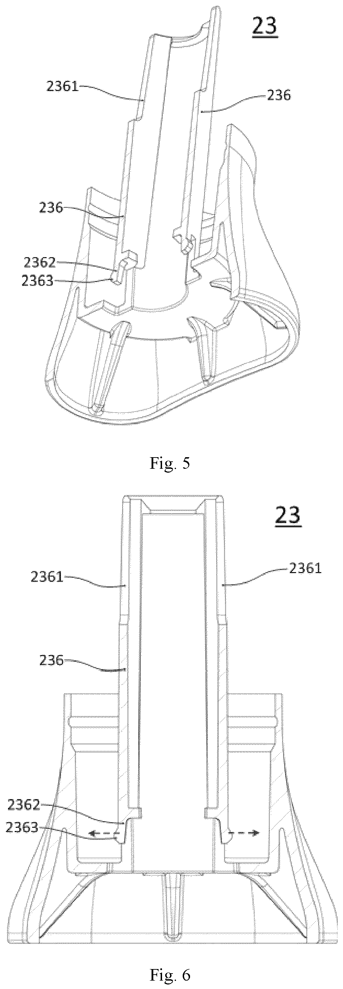

An upper part of a safety sleeve 236 is provided with a plurality of grooves 2361 as shown in Fig. 3. Referring to Figs. 5-6, the plurality of grooves 2361 are circumferentially arranged at an upper outer circle of the safety sleeve 236, so that the upper part of the safety sleeve 236 is in an open state, and is elastic. In assembly, when a needle is configured to connect with a sheath, a wall surface of the safety sleeve 236 can open outward for the sheath to enter. After entrance of the sheath, the safety sleeve 236 returns to a tube shape under the action of an elastic force of a material. The sheath is clamped by a sheath clamping structure, and the sheath of the needle can be removed along with an action of removing a pen cap 23.

### (1) Improvement of lower part of safety sleeve 236

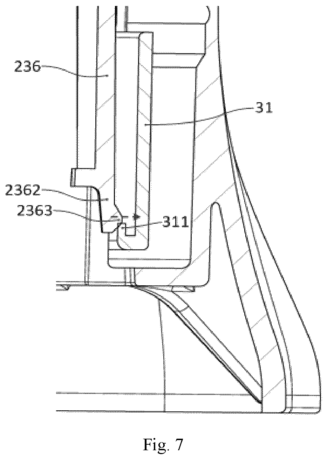

In an automatic injection pen in the Chinese Patent Application Publication No. 1 13499513A, a pen cap is fixed only by a connection of a clamping groove and a fifth protrusion of a pen tube body, and a lower part of the pen cap has no fixing structure, which has a risk of loose. Therefore, a lower part of the safety sleeve 236 of the present disclosure is provided with a fixing fastener structure. Referring to Figs. 5-6, the lower part of the safety sleeve 236 has a hollow cavity, and an elastic claw 2362 is provided in the hollow cavity. A fourth protrusion 2363 is provided below the elastic claw 2362. Preferably, the number of the elastic claw 2362 is two, and two elastic claws 2362 are arranged opposite to each other below the safety sleeve 236. The fourth protrusion 2363 is configured to protrude outward to exceed an outer surface of the safety sleeve 236. Upper part and lower parts of the fourth protrusion 2363 are configured to form a first guide slope. Referring to Fig. 7, a protruding sleeve 31 is provided below the needle cover 3, and a fifth protrusion 311 is inward provided on a bottom of a protruding sleeve 31 (the third protruding sleeve, as shown in Figs. 3-4, is in an unused state and excesses the pen tube body 21). In response to a case that the pen cap 23 is assembled with a pen body, the fifth protrusion 311 is configured to pass through a highest point above the two elastic claws 2362 to arrive at a position below the two elastic claws 2362. Then the two elastic claws 2362 are configured to be elastically reset, and to abut against the fifth protrusion 311. An upper part of the pen cap 23 is in mechanical limit by the pen tube body 21, and the lower part of the pen cap 23 is limited by the fifth protrusion 311. In an embodiment, the two elastic claws 2362 are configured to be compressed inward by force to apply an elastic force outward on the fifth protrusion 311, and the lower part of the two elastic claws 2362, under the action of a first slope on an outer surface of the fourth protrusion 2363, are configured to apply a downward component force on the fifth protrusion 311 to keep the automatic injection pen in the unused state as shown in Figs. 1-2 and to keep fixing of the pen cap 23. In response to a case that the pen cap 23 is removed, the fifth protrusion 311 is configured to pass through the highest point under the action of an external force to return to a free state, so that the needle cover 3 is separated from the pen cap 23.

### Embodiment 2 Improvement of locking-actuation mechanism

The locking-actuation mechanism includes an inner upper part of a pen tube stopper 22, the needle cover 3, an upper part of the piston assembly 4, a second spring 51 for drug administration and a spring cylinder 6.

### (1) Pen tube stopper 22

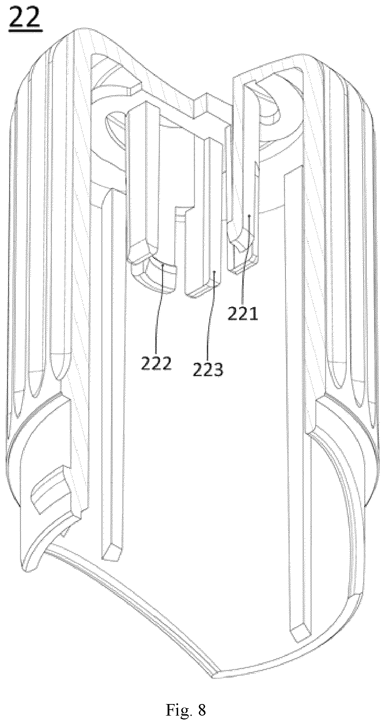

Referring to Fig. 8, an inner upper part of the pen tube stopper 22 is provided with a locking-limitation mechanism, including a locking claw 221. A bottom of the locking claw 221 is inwardly provided with a locking hook 222. The locking claw 221 is opposite provided. Two sides of the locking claw 221 are respectively provided with a limit claw 223, and the limit claw 223 is a straight blocking plate.

### (2) Needle cover 3

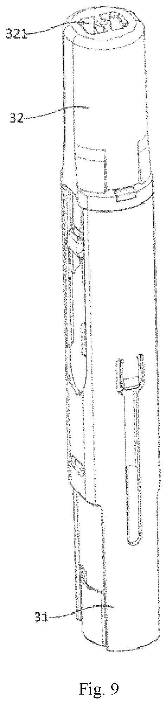

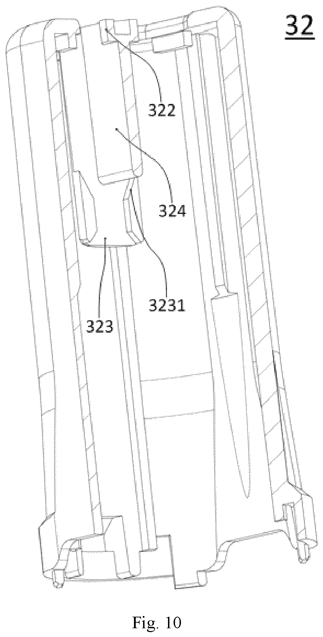

Referring to Fig. 9, the needle cover 3 adopts a split design owing to complex internal structure. The needle cover 3 includes a needle cover structure 31 and an actuation structure 32. The top of the needle cover 3 is provided with a fourth opening 321 similar to a first opening, and the fourth opening 321 is configured for a top of the piston assembly to pass through. An interior of the actuation structure 32, as shown in Fig. 10, includes a clamping column 322 and a guide plate 323.

The clamping column 322 is located between the fourth opening 321. In an embodiment, the number of the guide plate 323 is two, and two guide plates 323 are symmetrically arranged at two sides of the clamping column 322. An inner side of the guide plate 323 is provided with a second slope 3231. In an embodiment, the second slope 3231 is symmetrically provided on each of the two guide plates 323, and is symmetrically provided relative to the clamping column 322. A first connecting plate 324 is provided between the guide plate 323 on two sides of the clamping column 322 for connection reinforcement. A top of the first connecting plate 324 is connected with the clamping column 322 for the connection reinforcement.

### (3) Piston assembly 4

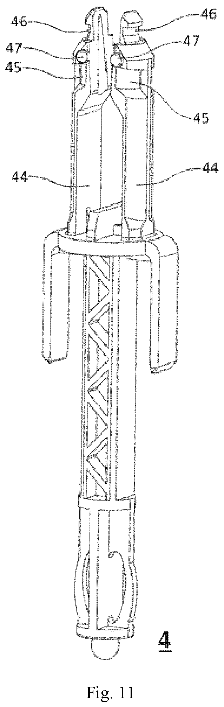

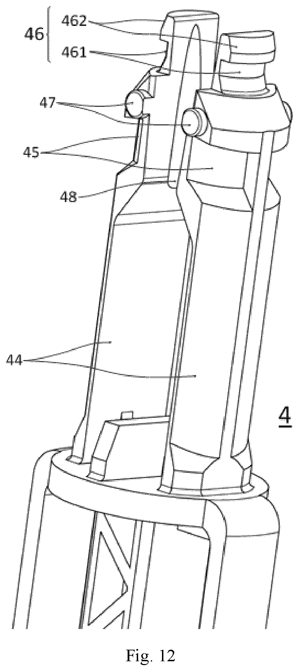

Referring to Figs. 11-12, the piston assembly 4 includes a push head, a push rod, and a withdrawal-triggering claw on a lower part of the piston assembly 4. A platform formed on an upper part of the push rod and an upper part of the withdrawal-triggering claw is provided with a spring support portion 44. The spring support portion 44 is a pair of elastic claws. An outer side of the spring support portion 44 is configured to accommodate the second spring 51. An upper part of the spring support portion 44 is provided with a locking-actuation structure.

The locking-actuation structure includes a spring locking groove 45, a piston locking groove 46 and an actuation column 47.

The piston locking groove 46 is configured to be clamped into the locking claw 221. In a case of non-use, the piston assembly 4 is locked and fixed in the shell of the automatic injection pen. The piston locking groove 46 includes a groove body 461, and the groove body 461 is clamped in the locking hook 222. An upper part of the groove body 461 is provided with a second guide slope 462 for assembly guiding.

In an embodiment, an upper inner part of the piston assembly 4 is provided with a guide groove 48, and the guide groove 48 is configured to fit the clamping column 322.

### (4) Spring cylinder 6

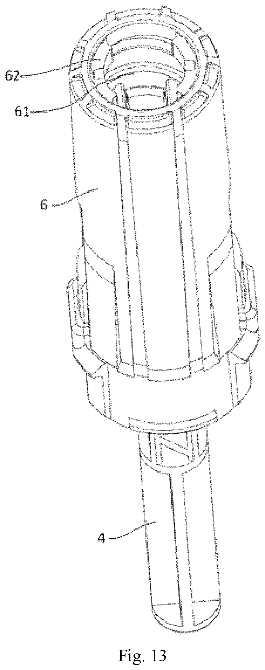

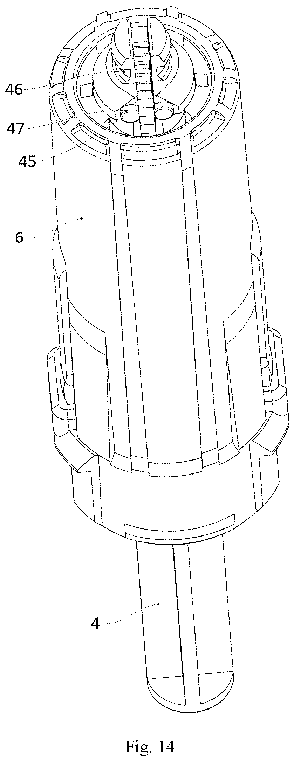

Referring to Figs. 13-14, the spring cylinder 6 is combined with the upper part of the piston assembly 4, and the second spring 51 is provided between the spring cylinder 6 and the piston assembly 4. A top of the spring cylinder 6 is provided with a piston actuation channel 61, and an inner side of the piston actuation channel 61 is provided with a spring locking structure 62. The upper part of the piston assembly 4 is configured to pass through the piston actuation channel 61, and the spring locking groove 45 is configured to be clamped in the spring locking structure 62 to realize clamping, so that the piston assembly 4 is connected with the spring cylinder 6.

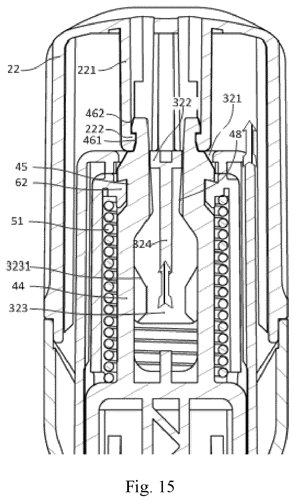

Referring to Fig. 15, which is an inner structural diagram of the automatic injection pen in a locking state, the automatic injection pen includes the piston assembly 4, the second spring 51, the spring cylinder 6, the needle cover 3 and the pen tube stopper 22 sequentially from inside to outside.

The spring support portion 44 of the piston assembly 4 is sleevedly provided with the second spring 51. An outer side of the second spring 51 is provided with the spring cylinder 6. An upper end of the second spring 51 is configured to abut against an inner side of the top of the spring cylinder 6, and a lower end of the second spring 51 is configured to abut against a bottom of the spring support portion 44.

The spring locking groove 45 of the spring support portion 44 is configured to be fastened with the spring locking structure 62 to combine the piston assembly 4 and the spring cylinder 6, so that the second spring 51 is kept compressed.

The upper part of the spring support portion 44 is configured to sequentially pass through the piston actuation channel 61 and the fourth opening 321, and insert into the locking-limitation mechanism of the pen tube stopper 22, so that the piston locking groove 46 is fastened with the locking claw 221. The piston assembly 4 is configured to be combined with the pen tube stopper 22, so that components in the needle cover 3 is fixed on a lower part of the pen tube stopper 22.

In an embodiment, in the locking state, the clamping column 322 is clamped in the spring support portion 44 and is kept in the guide groove 48, so that the upper part of the spring support portion 44 is maintained in an outward open state, so that two clamping connections between the clamping column 322 and the spring support portion 44 are kept in a reliable clamping state to avoid disconnecting, unlocking and actuation during a process of transportation and storage.

In this embodiment, the needle cover 3 is the only actuation component, and the needle cover 3 is configured to move upward to actuate.

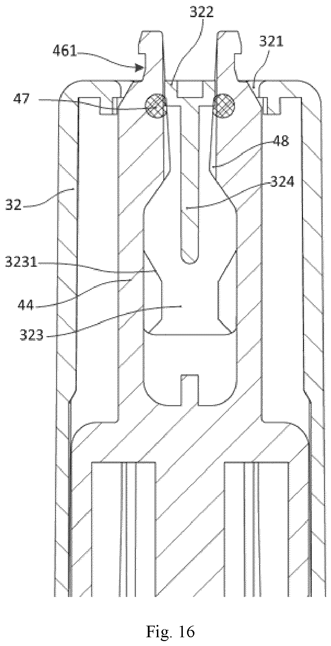

In an embodiment, referring to Figs. 16-18, an actuation process and principle of the needle cover 3 are as follows.

### (1) Early stage of actuation

During use of the automatic injection pen of the present disclosure, the bottom of the needle cover 3 is configured to abut against a part to be injected. Then the automatic injection pen is configured to be applied a downward force, so that the needle cover 3 moves upward relative to the shell and inner structure of the automatic injection pen. The clamping column 322 moves upwardly along the guide groove 48 until it completely moves out of the upper part of the spring support portion 44. The upper part of the spring support portion 44 can be compressed and deformed since it is not limited by the clamping column 322.

Two sides of the upper part of the spring support portion 44 are respectively provided with the actuation column 47 corresponding to the second slope 3231 of the guide plate 323. At this time, the guide plate 323 moves upwardly, so that the second slope 3231 is gradually closer to the actuation column 47.

### (2) Actuation process

The actuation column 47 is configured to be in contact with the second slope 3231, so that actuation column 47 drives the upper part of the spring support portion 44 to contract inwardly along the second slope 3231. In this process, the spring locking groove 45, the piston locking groove 46 are also compressed inwardly. Therefore, the spring locking groove 45 is separated from the spring locking structure 62, and the piston locking groove 46 is separated from the locking hook 222.

### (3) Actuation of automatic injection pen

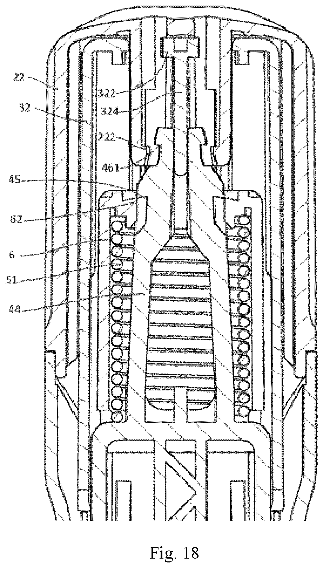

Referring to Fig. 18, the piston locking groove 46 is located on the upper part of the automatic injection pen and has a larger shrinkage, and will be separated from the locking hook 222 at the earliest, so that the spring support portion 44 will no longer be in contact with the pen tube stopper 22. Since the spring locking groove 45 is completely separated from the spring locking structure 62, the spring support portion 44 will no longer be fixed with the spring cylinder 6. The actuation column 47 is configured to move to a bottom of the second slope 3231 and to be separated from the second slope 3231, and the actuation column 47 is in a free state.

At this moment, a compression force of the second spring 51 is released to downwardly push the piston assembly 4 to perform actions, such as needling and injection.

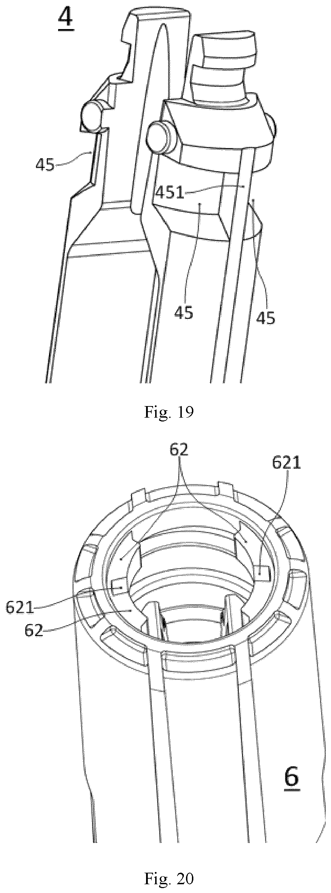

In an embodiment, referring to Fig. 19, a limit plate 451 is provided in the spring locking groove 45. Referring to Fig. 20, a middle of the spring locking structure 62 is provided with a limit groove 621. The limit plate 451 is configured to be clamped in the limit groove 621 in response to a case that the spring locking groove 45 is engaged with the spring locking structure 62, which can offset a circumferential rotating force and prevent the piston assembly 4 from rotating in the spring cylinder 6.

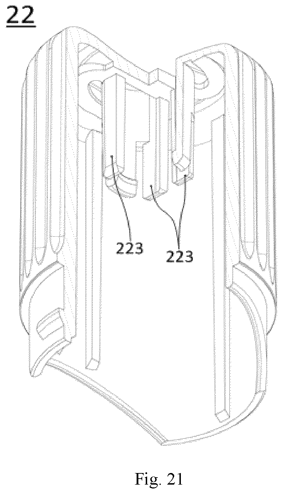

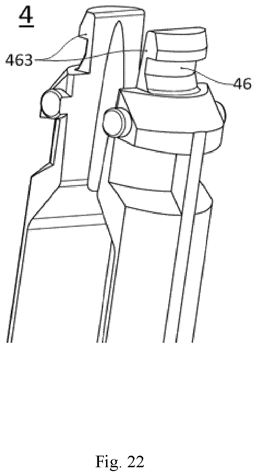

In an embodiment, referring to Fig. 21, a limit surface 463 is provided on two sides of the piston locking groove 46, and the limit surface 463 is configured to be clamped in the limit claw 223 as shown in Fig. 22. In response to a case of cooperation of the piston locking groove 46 and the locking hook 222, the limit claw 223 is configured to be blocked at the limit surface 463, which can offset the circumferential rotating force and prevent the piston assembly 4 from rotating in the pen tube stopper 22.

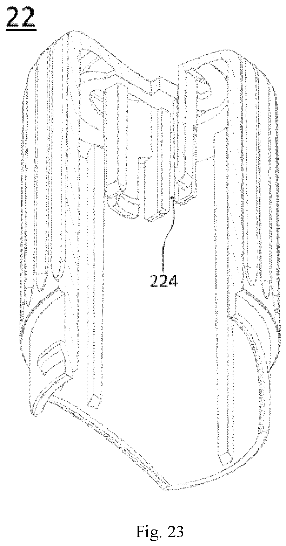

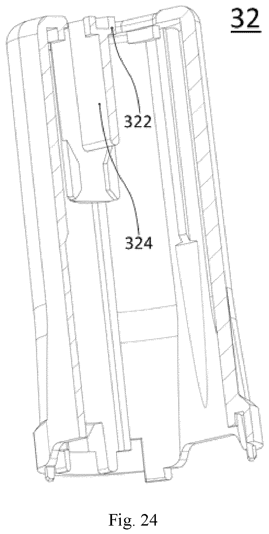

In an embodiment, referring to Fig. 23, a connecting plate channel 224 is provided at a middle of the limit claw 223. When the needle cover 3 moves upwardly due to a need of actuation, the clamping column 322, as shown in Fig. 24, is configured to enter a first cavity formed by the locking claw 221 and the limit claw 223, and the first connecting plate 324 is configured to insert into the connecting plate channel 224 provided in the middle of the limit claw 223 and pass through without barrier.

With the improvement of the structure of the locking-actuation mechanism, when the automatic injection pen of the present disclosure is not in use, the pen tube stopper 22, the spring cylinder 6 and the piston assembly 4 are connected together, which has reliable locking and avoids release of the second spring 51caused by mistake. The needle cover 3 moves upwardly for actuation, which is simple and easy to operate.

### Embodiment 3 Improvement of lower part of piston assembly 4

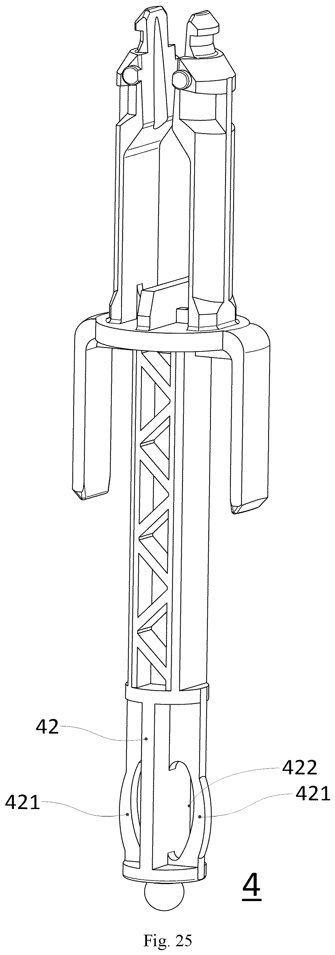

Referring to Fig. 25, a part of a push rod 42 the piston assembly 4 within a barrel 11 is provided with a third protrusion 421, and both sides of the piston assembly 4 are symmetrically provided with the third protrusion 421. In an embodiment, the third protrusion 421 is form by a material of the piston assembly 4 during injection molding. An outer diameter of the third protrusion 421 is larger than an inner diameter of the barrel 11. In an embodiment, the third protrusion 421 is elastic. The elasticity of the third protrusion 421 can form a second cavity 422 on an inner side of the third protrusion 421, so that the third protrusion 421 forms an elastic accommodation room.

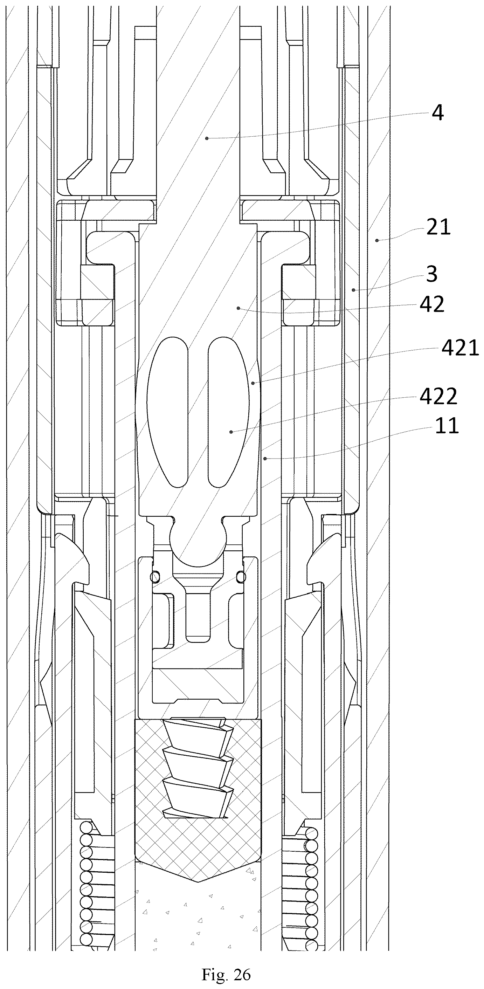

Referring to Fig. 26, when the lower part of the piston assembly 4 is installed into the barrel 11, the third protrusion 421 is configured to be compressed and deformed, and to abut against an inner wall of the barrel 11. Because the inner wall of the barrel 11 is smooth, the piston assembly 4 can still move in the barrel 11 under the action of the elastic force by release of the second spring 51 to realize injection administration. However, due to an additional friction between the third protrusion 421 and the barrel 11, the second spring 51 is configured to release to push the barrel 11 and the piston assembly 4 to move downwardly, so that the needle is inserted into an injection target, that is, the needling is performed first. Then the second spring 51 is configured to continue to release the elastic force, and to push the piston assembly 4 to move in the barrel 11 to perform the injection administration.

### Embodiment 4 Improvement of withdrawal mechanism

### (1) Delay of needle withdrawal

During release process of the second spring 51, the injection administration is realized, and after injection administration, the withdrawal-triggering claw is actuated to perform the needle withdrawal. In the prior art, the injection administration and the needle withdrawal are performed continuously. Even that a piston 111 which is configured to push a medicament just moves to an end of the barrel 11, the withdrawal-triggering claw of the piston assembly 4 immediately actuates a spring hook of the spring sleeve 8, otherwise there will be interference in a mechanical structure, causing that the medicament is injected unthoroughly, and the needle withdrawal after administration is performed without pause time. The medicament has not remained stably in human body and may be taken out with the action of the needle withdrawal. Therefore, it is necessary to carry out a corresponding structural improvement to delay the needle withdrawal.

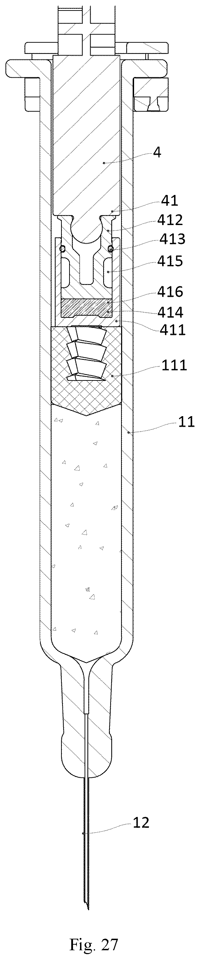

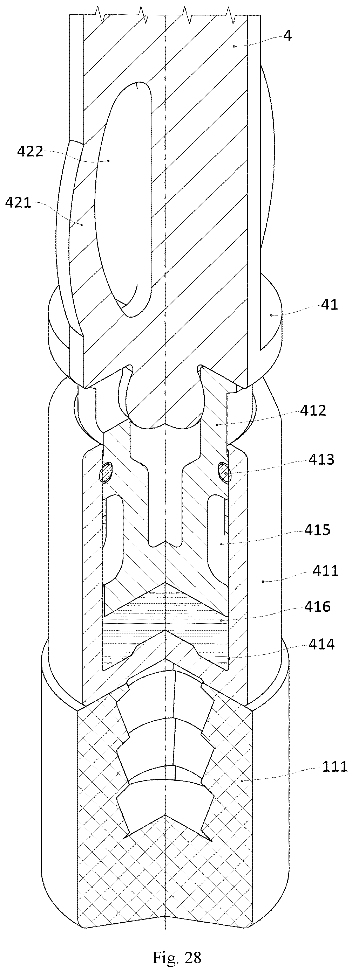

Referring to Figs. 27-28, a linear buffer is provided between the push head 41 of the piston assembly 4 and the piston 111 in the barrel 11.

The linear buffer includes a case 411 and a buffer plug 412, where a bottom of the case 411 is connected with the piston 111, and a top of the buffer plug 412 is connected with the push head 41.

A sealing ring 413 is provided between the case 411 and an upper part of the buffer plug 412 for slidable sealing. In an embodiment, the sealing herein is configured to discharge gas outward without causing significant liquid discharge. A first buffer cavity 414 is provided between a bottom of the buffer plug 412 and a bottom of an inner cavity of the case 411. A middle of the buffer plug 412 is provided with a recessed portion, and a second buffer cavity 415 is provided between the middle of the buffer plug 412 and an inner wall of the case 411. A connecting channel is provided between the first buffer cavity 414 and the second buffer cavity 415.

The first buffer cavity 414 is injected with a buffer fluid 416 before use. A viscosity of the buffer fluid 416 is higher than that of the medicament.

When the second spring 51 is released for the injection administration, a spring force is applied on the piston assembly 4 to produce a push force, and the push force is transmitted from the push head 41 to the buffer plug 412. The buffer plug 412 is configured to press the buffer fluid 416 in the first buffer cavity 414. Then the push force is transmitted to the case 411, and the piston 111 is pushed to move in the barrel 11 for administration. In this embodiment, the buffer fluid 416 slowly flows into the second buffer cavity 415 under the action of the push force. A flow speed of the buffer fluid 416 is slower than an injection speed of the medicament, therefore, the push force is mainly used for injection administration.

At a later stage of the release of the second spring 51, the medicament is injected thoroughly, the piston 111 is configured to abut against the end of the barrel 11. The spring force continues to be applied to push the buffer plug 412 to move, and the buffer fluid 416 completely flows from the first buffer cavity 414 to the second buffer cavity 415 until thorough discharge of the buffer fluid 416. In this embodiment, the withdrawal-triggering claw of the piston assembly 4 is in contact with the spring hook pf the spring sleeve 8 to open the spring hook, so that a first protrusion 52 is released to perform the needle withdrawal.

During a transfer process of the buffer fluid 416 from the 414 first buffer cavity 414 to the second buffer cavity 415, a gas in the second buffer cavity 415 is configured to be discharged outwardly through a sealing gap of the sealing ring 413, which can further produce an effect similar to air damping. When the buffer fluid 416 completely flows into the second buffer cavity 415, the buffer fluid 416 is difficult to leak through the sealing gap of the sealing ring 413 owing to its viscosity, so that components of the linear buffer are in a relatively stable state.

The linear buffer is provided. In this way, it can ensure that the medicament can be completely injected, and the needle withdrawal is performed after complete injection of the medicament, which forms a buffer period to delay the needle withdrawal, and meets a pause requirement after injection.

### (2) Improvement of actuation reliability of needle withdrawal

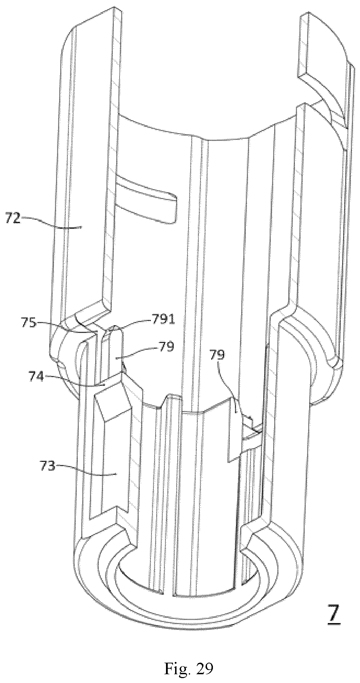

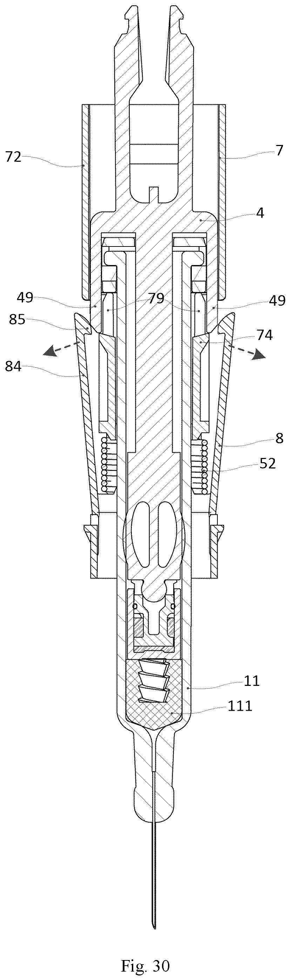

Referring to Fig. 29, a structure of a syringe sleeve 7 is locally improved. In an embodiment, a claw inner support 79 is provided in a third opening 75. The claw inner support 79 is connected with a first protrusion 74, which has a good structural rigidity. An outer surface of the claw inner support 79 is lower that that of the first protrusion 74. A top of the claw inner support 79 is provided with a third guide slope 791, and the third guide slope 791 is configured to guide an entry of the withdrawal-triggering claw 49. Referring to Fig. 30, the withdrawal-triggering claw 49 of the piston assembly 4 is configured to enter the third opening 75 and to be in contact with an inner side of the spring hook 85 of the spring sleeve 8, and the claw inner support 79 is configured to abut against an inner side of the withdrawal-triggering claw 49, so that the withdrawal-triggering claw 49 pushes the spring hook 85 outward to be separated from the first protrusion 74, and the first protrusion 52 is released. Each component begins to move along a longitudinal direction. Then the spring hook 85 elastically contracts and enters the sliding groove 73. The claw inner support 79 is provided, so that the withdrawal-triggering claw 49 is configured to be completely utilized to push the spring hook 85. That is, it can be avoided that the withdrawal-triggering claw 49 contracts inwardly (the withdrawal-triggering claw 49 has a cantilever structure) and cannot fully pushes the spring hook 85 to separate from the first protrusion 74.

### Embodiment 5 Improvement of connection structure between needle cover and spring sleeve

The needle cover 3 is configured to be in snap-fit with the spring sleeve 8. In an original design, a fixing clamp provided on the spring sleeve 8 is a straight snap fit, which can only be installed through an elastic deformation of a material, and needs to apply a relative large force during an installation process.

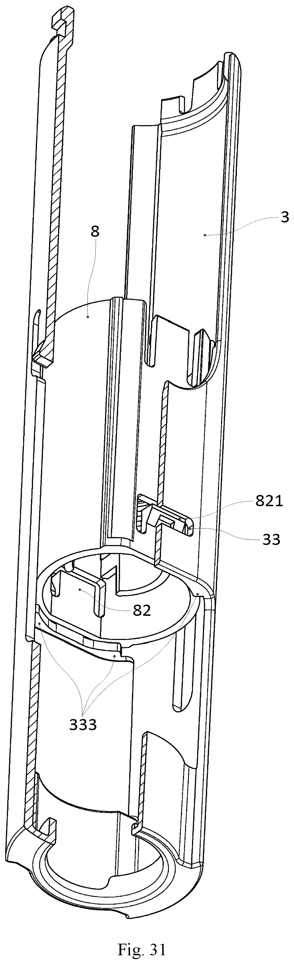

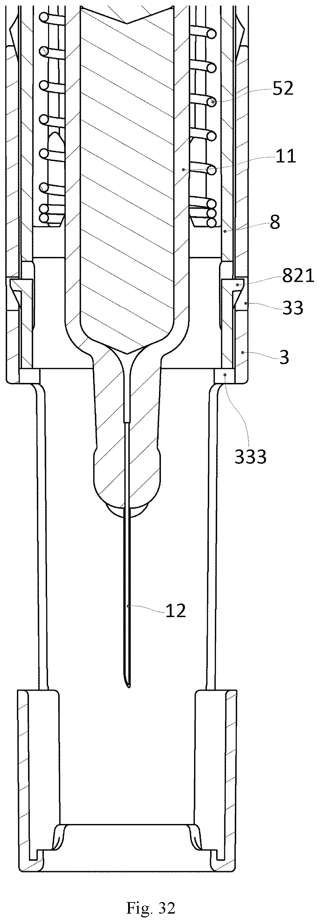

Referring to Figs. 31-32, an improved connection structure is shown. The spring sleeve 8 is provided with an elastic fixing fastener 82. Three sides of the elastic fixing fastener 82 are hollow, and a bottom of the elastic fixing fastener 82 is connected with a main body of the spring sleeve 8. An upper part of the elastic fixing fastener 82 is configured as a support and limit part 821, and is configured to extend into a fifth opening 33 provided at the needle cover 3 in a natural state. A fourth guide slope is downward and inward provided below the support and limit part 821. The needle cover 3 is provided with a base support 333.

When the spring sleeve 8 is installed in the needle cover 3, the spring sleeve 8 is inserted through the top of the needle cover 3. The fourth guide slope of the elastic fixing fastener 82 is gradually in contact with the needle cover 3, so that the elastic fixing fastener 82 contracts inwardly under the action of a force until ejection when it reaches the fixing opening 33. The bottom of the spring sleeve 8 just abuts against the base support 333. In this way, a reliable installation of the spring sleeve 8 in the needle cover 3 is realized. In addition, upper and lower directions of the spring sleeve 8 can bear force without sliding and separation.

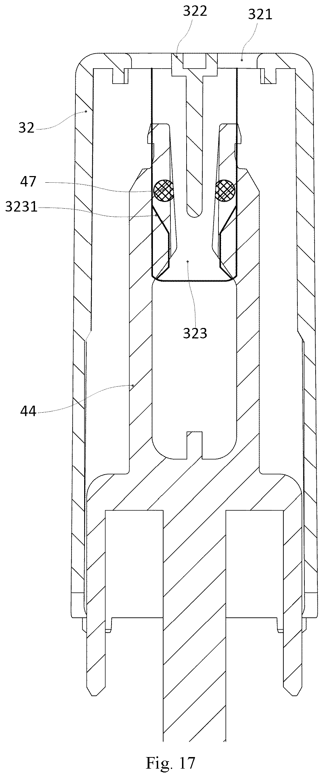

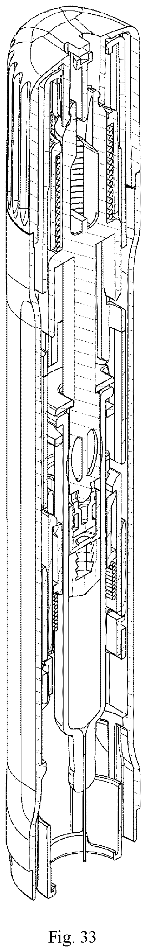

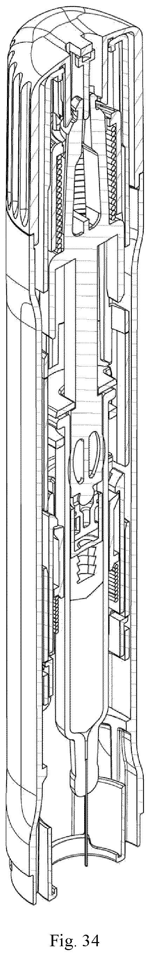

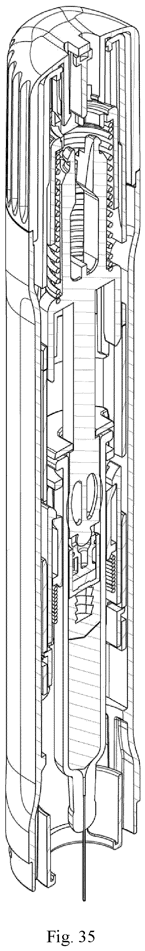

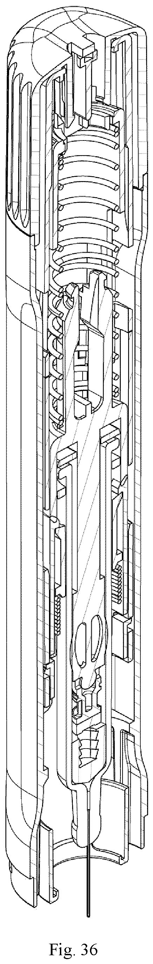

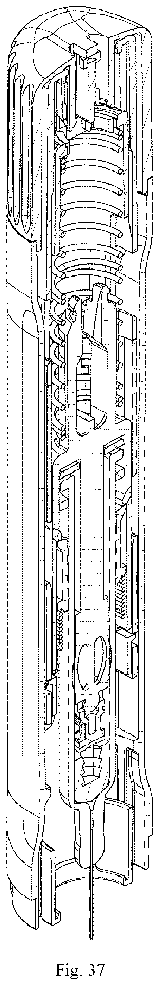

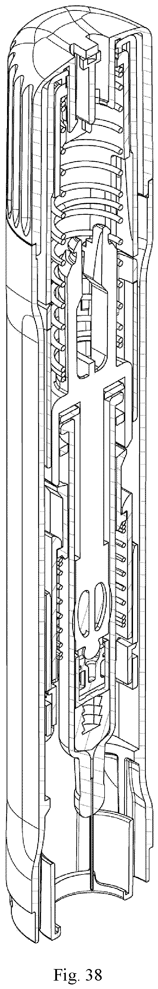

A use process of the automatic injection pen of the present disclosure is shown in Figs. 1-4 and 33-38.
(1) Figs. 1-2 show the automatic injection pen in the unused state.
(2) Fig. 3 shows the automatic injection pen ready for use in a process of removing of the pen cap.
(3) The second spring is in a compressed state, and the first protrusion is in a releasing state before use.

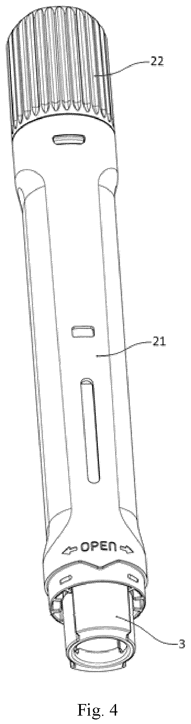

(4) Fig. 4 shows the automatic injection pen ready for use with removal of the pen cap. A user holds the pen tube body 21, then press the bottom of the needle cover 3 on a skin of the part to be injected, and then presses down so that the pen tube body 21 moved down and abuts against the skin of the part to be injected. Then, the automatic injection pen is actuated and starts to an automatic injection operation.
(5) Referring to Fig. 33, the needle cover 3 relatively moves upward to actuate the locking-actuation mechanism. Further reference can be seen in Fig. 17, with movement of the needle cover 3, the first protrusion is compressed.
(6) Referring to Fig. 34, the spring support portion contracts inwardly and is no longer in contact with the pen tube stopper. The second spring is in the free state and starts to release the elastic force. Further reference can be seen in Fig. 18, the spring hook completely hooks the first protrusion. The first protrusion is compressed to store energy.
(7) Referring to Fig. 35, the first protrusion is partially released. Under the action of the friction between the third protrusion of a lower part of the push rod and the inner wall of the barrel, a syringe is pushed downwardly, especially the needle is pushed out and inserted into the part to be injected.
(8) Referring to Fig. 36, the second spring continues to be released and overcomes the friction between the third protrusion of the lower part of the push rod and the inner wall of the barrel, so that the medicament in the syringe is injected into a patient.
(9) Referring to Fig. 37, when the second spring is about to be completed, the medicament in the syringe is completely injected into the patient. Then the linear buffer starts to work. Then the withdrawal-triggering claw enters the third opening and push the spring hook outward.
(10) Referring to Fig. 38, the first protrusion is released to perform the needle withdrawal, and the components, such as syringe, move upwardly as a whole. Finally, the needle is completely hidden in the needle cover to complete the injection and the needle withdrawal.

Embodiments described above are only preferred embodiments of the present disclosure, which are not intended to limit the scope of the present disclosure. Various transformations and replacements made by those of ordinary skill in the art without departing from the spirit of the present disclosure shall fall within the scope of the disclosure defined by the appended claims.

## Claims

1. An automatic injection pen, comprising:
a shell;
a needle cover;
an inner sleeve; and
a syringe assembly;
**characterized in that** the needle cover is slidably provided in the shell; a top of the needle cover is provided with a first opening; an inner side of a lower part of the needle cover is fixedly provided with a spring sleeve; an upper part of the spring sleeve is provided with a spring hook configured to protrude inwardly; the inner sleeve is slidably arranged in the needle cover; a top of the inner sleeve is provided with a second opening; an outer side of a lower part of the inner sleeve is provided with a sliding groove; a bottom of the sliding groove is closed, and an upper part of the sliding groove is provided with a first protrusion; the spring hook is configured to be clamped in the sliding groove; and a first spring is provided between the lower part of the inner sleeve and the spring sleeve for needle withdrawal;
the syringe assembly comprises a barrel, a needle, and a piston assembly; the barrel is arranged in the inner sleeve; the needle is arranged at a bottom of the barrel, and the piston assembly is provided in an upper part of the barrel; the piston assembly comprises a push head, a push rod, a spring support portion, a second protrusion, a guiding portion and an actuation portion sequentially from bottom to top; an outer side of the push rod is provided with a withdrawal-triggering claw; the second protrusion is configured to be pre-clamped in the second opening; an outer side of the spring support portion is provided with a second spring for drug administration; the second spring is configured to be pre-compressed to abut against an inner side of the top of the inner sleeve; the guiding portion is configured to pass through the first opening; the lower part of the inner sleeve is provided with a third opening configured for the withdrawal-triggering claw to pass through; and the withdrawal-triggering claw is located above the third opening, and is located above the spring hook;
the automatic injection pen further comprises a spring cylinder and a pen tube stopper; and the piston assembly, the second spring, the spring cylinder, the needle cover, and the pen tube stopper constitute a locking-actuation mechanism;
an upper part of the piston assembly comprises the spring support portion; the spring support portion is a pair of elastic claws; an outer side of the spring support portion is configured to accommodate the second spring; and an upper part of the spring support portion is provided with a locking-actuation structure;
the locking-actuation structure comprises a spring locking groove, a piston locking groove and an actuation column;
the spring locking groove is located on a middle-upper part of the spring support portion; the piston locking groove is located on the upper part of the spring support portion; and the actuation column is located on an outer side of the middle-upper part of the spring support portion;
the second spring is a compression spring; and the spring cylinder is provided on an outer side of the second spring;
an upper end of the second spring abuts against an inner side of a top of the spring cylinder, and a lower end of the second spring abuts against a bottom platform of the spring support portion;
the top of the spring cylinder is provided with a piston actuation channel; and an inner side of the piston actuation channel is provided with a spring locking structure;
the needle cover is sleeved outside the spring cylinder, and is configured to axially move;
the top of the needle cover is provided with a fourth opening configured for a top of the piston assembly to pass through; and an inner side of the needle cover is provided with a guide plate;
an inner side of the guide plate is provided with a slope;
an outer side of the needle cover is provided with the pen tube stopper;
an interior of the pen tube stopper is provided with a locking claw; and a bottom of the locking claw is inwardly provided with a locking hook;
the locking-actuation mechanism is configured to have a locking state and an actuation state;
in response to the locking state of the locking-actuation mechanism:
the upper part of the spring support portion is configured to pass through the piston actuation channel and the fourth opening; the piston locking groove is configured to be clamped into the locking claw, and to be engaged with the locking hook;
the middle-upper part of the spring support portion is configured to be clamped on the top of the spring cylinder; the spring locking groove is configured to be clamped in the spring locking structure;
the second spring is in a limited compression state; and
the needle cover is located at a position corresponding to a lower part of the shell; the guide plate is located on the outer side of the spring support portion; and the slope is located below the actuation column;
in response to the actuation state of the locking-actuation mechanism:
the needle cover is located at a position corresponding to an upper part of the shell; the guide plate is configured to move upward along the outer side of the spring support portion; the slope is in contact with the actuation column; the actuation column is configured to be guided to move inward to a bottom free space of the slope to drive the upper part of the spring support portion to contract inward;
the piston locking groove is configured to move inward to be separated from the locking hook;
the spring locking groove is configured to move inward to be separated from the spring locking structure; and
the second spring is in an elastic release state;
a part of the push rod within the barrel is provided with a third protrusion; and both sides of the piston assembly are symmetrically provided with the third protrusion;
a linear buffer is provided between the push head of the piston assembly and a piston in the barrel;
an inner support for the withdrawal-triggering claw is provided in the third opening; and the inner support is connected with the first protrusion; and
an outer surface of the inner support is below an outer surface of the first protrusion.

2. The automatic injection pen of claim 1, **characterized in that** the number of the guide plate is two; two guide plates are symmetrically arranged at an inner bottom of the fourth opening;
the number of the slope is four, and each of the two guide plates is symmetrically provided with two of four slopes;
the number of the actuation column is four; the number of the spring support portion is two, and two spring support portions are respectively provided at two sides of the piston assembly; and four actuation columns are respectively located on both sides of the two spring support portions; and
the four actuation columns are respectively configured to cooperate with the four slopes.

3. The automatic injection pen of claim 1, **characterized in that** a clamping column is provided in the fourth opening; and the number of the spring support portion is two, and two spring support portions are respectively provided at two sides of the piston assembly;
in a case that the locking-actuation mechanism is in the locking state, the clamping column is located between the spring support portions; and
in a case that the locking-actuation mechanism is in the actuation state, the clamping column is located above the two spring support portions.

4. The automatic injection pen of claim 3, **characterized in that** a middle inner side of an upper part of each of the two spring support portions is provided with a guide groove; and
the guide groove is configured to fit the clamping column.

5. The automatic injection pen of claim 1, **characterized in that** a limit claw is provided on an outer side of the locking claw; and the limit claw is a straight blocking plate;
two sides of the piston locking groove are each provided with a limit surface; and
the limit surface is configured to fit the limit claw.

6. The automatic injection pen of claim 1, **characterized in that** a limit plate is provided in the spring locking groove; and a middle of the spring locking structure is provided with a limit groove; and
the limit plate is configured to be clamped into the limit groove in response to a case that the spring locking groove is engaged with the spring locking structure.

7. The automatic injection pen of claim 1, **characterized in that** the third protrusion and the piston assembly are integrally formed;
an outer diameter of the third protrusion is larger than an inner diameter of the barrel; and
an inner side of the third protrusion is provided with a cavity; and the third protrusion has a transverse elasticity.

8. The automatic injection pen of claim 1, **characterized in that** the linear buffer comprises a case and a buffer plug;
a bottom of the case is connected with the piston; and a top of the buffer plug is connected with the push head;
a sealing ring is provided between the case and an upper part of the buffer plug for slidable sealing; and
a first buffer cavity is provided between a bottom of the buffer plug and a bottom of an inner cavity of the case;
a middle of the buffer plug is provided with a recessed portion; and a second buffer cavity is provided between the recessed portion and an inner wall of the case;
a connecting channel is provided between the first buffer cavity and the second buffer cavity; and
the first buffer cavity is configured to be injected with a buffer fluid before use.

9. The automatic injection pen of claim 1, further comprising:
a pen cap;
wherein an inner side of the pen cap is provided with a safety sleeve; a plurality of grooves are circumferentially arranged at an upper part of the safety sleeve; and
the upper part of the safety sleeve is in an open state, and is elastic.

10. The automatic injection pen of claim 1, further comprising:
a pen cap;
wherein an inner side of the pen cap is provided with a safety sleeve; and a lower part of the safety sleeve is provided with a fixing fastener structure;
the lower part of the safety sleeve has a hollow cavity; an elastic claw is provided in the hollow cavity; and a fourth protrusion is provided below the elastic claw;
the number of the elastic claw is two; and two elastic claws are arranged opposite to each other below the safety sleeve;
the fourth protrusion is configured to protrude outward to exceed an outer surface of the safety sleeve;
upper and lower parts of the fourth protrusion are configured to form a guide slope;
a protruding sleeve is provided below the needle cover; and a fifth protrusion is inward provided on a bottom of the protruding sleeve; and
in response to a case that the pen cap is assembled with a pen body, the fifth protrusion is configured to pass through a highest point above the two elastic claws to arrive at a position below the two elastic claws; and the two elastic claws are configured to be elastically reset, and to abut against the fifth protrusion.

11. The automatic injection pen of claim 1, **characterized in that** the spring sleeve is provided with an elastic fixing fastener; three sides of the elastic fixing fastener are hollow; and a bottom of the elastic fixing fastener is connected with a main body of the spring sleeve;
an upper part of the elastic fixing fastener is configured as a support and limit part, and is configured to extend into a fifth opening provided at the needle cover in a natural state; and the fifth opening is configured to fix the spring sleeve;
a guide slope is downward and inward provided below the support and limit part; and
the needle cover is provided with a base support.
